# EUROPEAN PATENT APPLICATION

(11) **EP 3 795 073 A1**
(43) Date of publication of application: **24.03.2021**
(21) Application number: 19198090.3
(22) Date of filing: 18.09.2019
(51) Int. Cl.: A61B 5/042, A61N 1/39

(54) **SENSOR DEVICE, SYSTEM, AND METHOD FOR MONITORING A CARDIAC ACTIVITY**

(71) Applicant: BIOTRONIK SE & Co. KG, 12359 Berlin (DE)
(72) Inventor: Rump, Jens, 12049 Berlin (DE); van Ooyen, André, 10717 Berlin (DE)
(74) Representative: Galander, Marcus

(57) **Abstract**

A sensor device (100) adapted to be implanted in the gastric fundus (501) of a patient is presented. The sensor device (100) comprises: a fixing element (101) configured for fixing the sensor device (1) to the stomach wall (500, 502); a contact element (101, 102) adapted to be arranged in contact with the stomach wall (500, 502) and to receive electricals signals from the patient's heart (400) via the stomach wall; a digital processing device (106) configured for processing the received electrical signals (400); and a communication system (103) configured for wirelessly transmitting signals to a receiver device in dependence on the processed electrical signal.

## Description

The instant invention relates to a sensor device that may be used for monitoring a cardiac activity of a patient. Further, the instant invention relates to a system comprising such a sensor device, as well as to a method for monitoring a cardiac activity by means of such a sensor device.

Implants for continuous monitoring of heart activity are usually implanted subcutaneously in the shoulder or chest region of the patient, similarly to pacemakers or implantable defibrillators. Although the surgical intervention may be carried out in a minimally invasive way, the required skin incision and the formation of a pocket in the fat tissue or connective tissue are associated with a risk of infection. In addition, the finite lifespan the implant battery makes it necessary to replace the implant by carrying out further surgical interventions at regular intervals.

It is an object of the present invention to provide for a sensor device that reliably allows continuous monitoring of heart activity while overcoming the abovementioned disadvantages of known solutions. Further, a corresponding system and method shall be provided.

According to a first aspect, a sensor device is provided that is adapted to be implanted in the gastric fundus of a patient. The sensor device comprises: a fixing element configured for fixing the sensor device to the stomach wall; a contact element adapted to be arranged in contact with the stomach wall and to receive (i. e., detect) electrical signals from the patient's heart via the stomach wall; a digital processing device configured for processing the received electrical signals; and a communication system configured for wirelessly transmitting signals to a receiver device in dependence on the processed electrical signal.

The receiver device may be another implantable device, e.g. a looprecorder, a pacemaker (e.g. a leadless pacemaker), an ICD (ICD - implantable cardioverter-defibrillator) or a sICD (sICD - subcutaneous implantable cardioverter-defibrillator). Alternatively, the receiver device may be a device located external of the patient's body, e.g. a patient device like a smartphone or a tablet, a clinician device like a smartphone or a tablet, or a programmer.

According to a second aspect, a system is presented. The system comprises a subcutaneous implantable cardioverter-defibrillator (sICD) and a sensor device according to the first aspect. The sensor device is configured for delivering electrical pulses to the stomach wall in dependence on an sICD pulse delivered by the sICD, wherein the electrical pulses are delivered by the sensor device via the contact element and/or via a further contact element that is adapted to be arranged in contact with the stomach wall.

According to a third aspect, a method for monitoring a cardiac activity of a patient comprises: providing a sensor device according to the first aspect; implanting the sensor device in the gastric fundus of the patient via the esophagus; and receiving, by means of a receiver device, signals transmitted by the communication system, wherein the transmitted signals are indicative of the cardiac activity. For the receiver device, the explanations provided above apply.

Hence, it is proposed to implant a sensor in the abdomen by fixing it to the stomach wall and to monitor the cardiac activity via the stomach wall. The invention is based on the insight that an alternative suitable position of a sensor for the detection of cardiac excitation signals is the cranial stomach wall at the gastric fundus. An advantage of this sensor position is the non-invasive accessibility via the esophagus and the short distance to the apex of the heart.

By choosing a location for the sensor that is accessible through natural orifices (namely, via the esophagus), the abovementioned risks associated with skin incisions etc. may be avoided. Moreover, a relatively uncomplicated placement of the sensor device under visual control (comparable to a gastroscopy) is possible. When a battery has to be replaced, the battery and/or the sensor as a whole can be replaced very quickly and safely.

Compared to cardiac implants, such as electrodes for the therapy of bradycardia or tachycardia, or pacemakers, which are anchored directly in the heart wall without electrodes, there are fewer restrictions on the weight and form factor of the sensor device. The mechanical loads in the gastric fundus are relatively low because there is less movement compared to the heart.

In the following, features of some exemplary embodiments are described.

For example, the digital processing device may be coupled to the contact element and may be configured for digitizing the electrical signals received from the contact element. The digital processing unit may further be configured for storing digital data indicative of the received electrical signals.

The communication system may comprise a transmitter unit for wirelessly transmitting the digital data, e. g., to a receiver device. For example, the data transmission may be based on a radio frequency (RF) communication link.

The communication system may also comprise a receiver unit, such that the sensor may be configured for receiving communication, e.g. from another implantable device or from outside the patient's body, e. g., for the purpose of control or (re-)programming of the sensor device.

The contact element provides a contact surface for contacting the stomach wall. Thus, the contact element may form a (metallic) contact electrode for receiving electrical signals from the patient's heart. For example a contact element in the form of an annular metal surface may be brought in direct contact with the stomach wall.

In an embodiment, the fixing element comprises at least a screw, such as a helical screw. The screw may be fixed to a housing of the sensor device.

In a variant, the fixing element (e. g, in the form of a screw) may at the same time serve as an electrode pole (i. e., as a contact element or a part thereof) for receiving electrical signals from the heart via the stomach wall and/or for delivering electrical pulses to the heart via the stomach wall.

In a further embodiment, the fixing element comprises at least one hook. For example, the hook may comprise or consist of a shape memory material, such as nitinol. The fixing element may thus be configured for forming an arch, i. e., a hook shape, when it is pushed out of a lumen, such as a working channel of a gastroscope, for example. Conversely, the hook-shaped fixing element may be retractable into a lumen, thereby assuming a more elongate shape.

In a variant, the fixing element is arranged at an end of an elongate electrode element, e. g. in the form a screw. For example, the elongate electrode element may form part of the communication system and may be used as a monopolar antenna (or a part thereof).

Further, in an embodiment, the sensor device comprises a hermetically sealed housing, wherein the digital processing device (such as a processor unit and a memory unit) and at least a part of the communication system (such as transmitter and/or receiver unit for unidirectional or bidirectional communication) are arranged inside the housing. The housing may further contain a battery for the power supply of the sensor device.

In an embodiment, the housing comprises or consists of titanium or another biocompatible material, such as PEEK, polyurethane, platinum, silicone, tantalum, or MP35N.

The hermetically sealed housing of the sensor device may have a cylindrical or cuboid shape. A maximum outside diameter transverse to a longitudinal axis may be in the range from 3 mm to 15 mm, for example. The sensor device may thus be suitable to be guided through a working channel of a commercial gastroscope or to be attached distally to a gastroscope without increasing the outside diameter of the gastroscope arrangement.

The fixing element may be arranged at one end of such a cylindrical or cuboid housing. The entire housing of the sensor device or part of the housing may also be used as a support to the fixation.

In an embodiment, the fixing element is a rotatable screw extending at least partially in a lumen provided in the sensor device. For example, the screw may be rotated (screwed) by means of a screw mandrin so as to protrude from the housing and engage with the stomach wall when the sensor device has reached a suitable position. To this end, the screw mandrin may be inserted in the lumen where it may engage with the rotatable screw.

The lumen and screw may be guided centrally or decentrally within the housing or may be arranged externally on the housing. In the case of a decentralized screw fixation, for example, a metal surface being arranged next to the fixing screw can be used as a second electrode pole for the detection of the cardiac excitation signals.

At one end of the housing, for example, a rigid loop may be attached, with the help of which the sensor may be rotated for fixing and/or for facilitating a replacement of a battery.

The communication system may comprise a communication antenna structure. In an embodiment, the communication system comprises at least one of a loop antenna, a dipolar antenna, and a monopolar antenna. For example, the antenna be arranged in the form of one or more (bipolar) loop(s) at an end of the housing that is in contact with the stomach wall or at another end of the housing, e. g., opposite to the end that is in contact with the stomach wall.

In an embodiment, the at least one antenna is embedded in a protective plastic sheath. The protective plastic sheath may be flexible. For example, the protective plastic sheath may comprise of consist of silicone or of another flexible plastic.

Further, in a variant embodiment, the sensor device is configured for assuming a transport configuration, in which the antenna and the protective plastic sheath are folded over the fixing element. Thus, the risk of injury, e. g., of the esophagus, during implantation may be minimized. This may be particularly advantageous in embodiments wherein the fixing element is a fixed screw protruding from the housing.

It is also within the scope of the invention that the sensor device is configured for delivering electrical pulses to the stomach wall via the contact element and/or a further contact element which is adapted to be arranged in contact with the stomach wall (and which may be dedicated for this purpose). The electrical pulses delivered by the sensor device may be, for example, pacing pulses for regular stimulation of the heart, higher-energy antitachycardia pulses (ATP) or pulses provided as an after-shock pacing.

For example, in the context of a system according to the second aspect, the sensor device may be activated by the sICD via a communication link. Hence, a communication link, such as a wireless communication link may be provided between the sICD and the sensor device.

The communication via the communication link may take place electromagnetically and may involve the (e. g. RF based) communication system of the sensor device. Other possibilities are acoustic (ultrasound) or galvanic signal transmission.

In an embodiment of the third aspect, the sensor device is implanted by means of a gastroscope. Commercially available gastroscopes for diagnosis or interventions usually have a light source, a camera or a CCD sensor, and one or more working channels. The working channels may have a diameter in the range from 2 mm to 15 mm. The outside diameter of the gastroscope is usually in the range from 5 mm to 20 mm.

In a variant embodiment, the sensor device is arranged inside a working channel of the gastroscope when passing through the esophagus. In particular, the sensor device may be entirely contained in the working channel during this phase of the implantation. This variant may be appropriate especially in case the fixing element is a fixed screw protruding from the housing of the sensor device, because injuries of the esophagus may thus be prevented.

For example, a diameter of the sensor device may be in the range from 3 mm to 5 mm, so that the sensor device can be readily pushed through the working channel. When the sensor device has reached a suitable position in the gastric fundus, it may be fastened by means of the fixing element, e. g. by rotating the sensor device, thereby screwing the fixing element into the stomach wall.

Alternatively, the sensor device may be implanted by means of a gastroscope, wherein the sensor device is attached at a distal end of the gastroscope when passing through the esophagus. This is to say that the sensor device may also be arranged external of the working channel(s) of the gastroscope during this phase of the implantation. In this case, a larger diameter of the sensor device, e. g. similar to the outer diameter of the gastroscope, is possible. The attachment of the sensor device outside the gastroscope may be effected using a clamping instrument (such as a biopsy forceps) inside the working channel. For example, the clamping instrument may be connected to a rigid loop arranged on the housing of the sensor device.

In a further embodiment of the third aspect, the method comprises: providing a system according to the second aspect; implanting each of the sensor device and the sICD in the patient's body, wherein the sensor device is implanted in the gastric fundus via the esophagus; delivering one or more sICD pulses by means of the sICD; and delivering one or more electrical pulses to the stomach wall by means of the sensor device in dependence on the one or more sICD pulses delivered by the sICD.

For example, the sensor device detects an electric field resulting from the one or more sICD pulses and delivers the one or more electrical pulses in reaction to the detecting the electric field.

Additionally or alternatively, the sICD may inform the sensor device via a communication link about the delivery of the one or more sICD pulses and the sensor device may deliver the one or more electrical pulses in reaction to being informed about the delivery of the one or more sICD pulses via the communication link.

Thus, the sICD may inform the sensor device when a shock has been triggered and, as a reaction, the sensor device may start a stimulation. Alternatively or additionally, the sensor device may detect the strongly increased electric fields that arise during a shock and may then initiate an after-shock pacing on its own.

All aspects and features of the embodiments described above and in the following can be combined with each other unless explicitly stated otherwise.

In particular, the method according to the third aspect may use a sensor device or a system according to the first and second aspects of the invention. Hence, what is stated above and in the following regarding features and variants of the sensor device and the system of the first and second aspects may analogously apply to the method of the third aspect, and vice versa.

The various features and advantages of the present invention may be more readily understood with reference to the following detailed description and the embodiments shown in the drawings. Herein,
- Fig. 1: schematically and exemplarily illustrates a sensor device implanted in the gastric fundus of a patient;
- Figs. 2A-B: each schematically and exemplarily illustrate a sensor device in accordance with one or more embodiments;
- Figs. 3A-B: each schematically and exemplarily illustrate a sensor device in accordance with one or more embodiments;
- Figs. 4A-B: each schematically and exemplarily illustrate a sensor device in accordance with one or more embodiments;
- Figs. 5A-D: each schematically and exemplarily illustrate a section of a gastroscope suitable for implanting a sensor device in accordance with one or more embodiments; and
- Figs. 6: schematically and exemplarily illustrates a system in accordance with one or more embodiments.

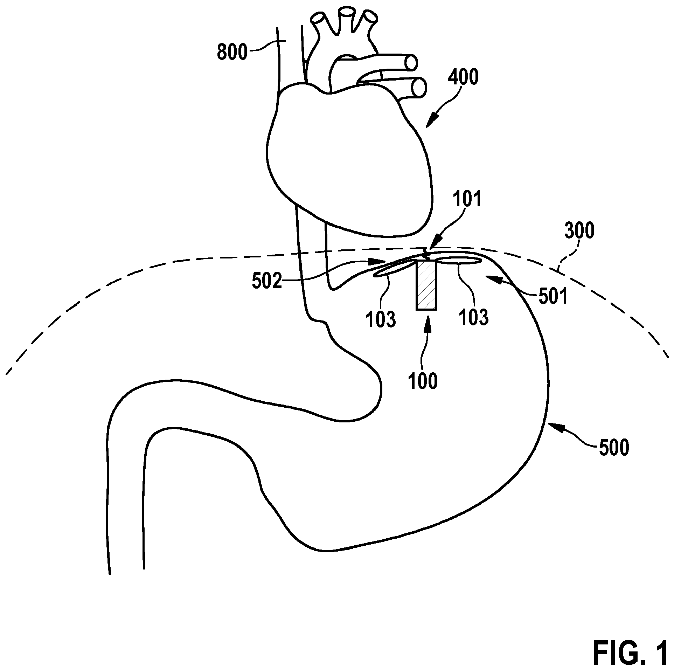

Fig. 1 schematically and exemplarily shows a sensor device 100 that is implanted in the gastric fundus 501 of a patient. The sensor device 100 is fixed to the stomach wall 500 and more precisely to the gastric fornix 502 by means of a fixing element 101, e.g., in the form of a screw 101. The sensor device 100 is situated close to the apex of the patient's heart 400 so that it may be used to detect electrical signals stemming from the beating heart 400 and monitor the cardiac activity.

The detected heart signals may be digitized within the sensor device 100. The acquired data on the heart activity may then be wirelessly transmitted to a receiver device (not illustrated) that is arranged external of the patient's body. To this end, the sensor device 100 comprises a communication system 103, such as a RF based communication system including a transmitter unit and optionally also a receiver unit. In the exemplary embodiment illustrated in Fig. 1, the communication system 103 also comprises two loop antennas 103 for enabling the wireless communication.

For example, the sensor device 100 may have been implanted via the esophagus 800, e. g., by means of a gastroscope 200. This will be explained in more detail further below.

Fig. 1 schematically depicts a cross-section of the thoracic diaphragm 300 extending between the heart 400 and the stomach 500.

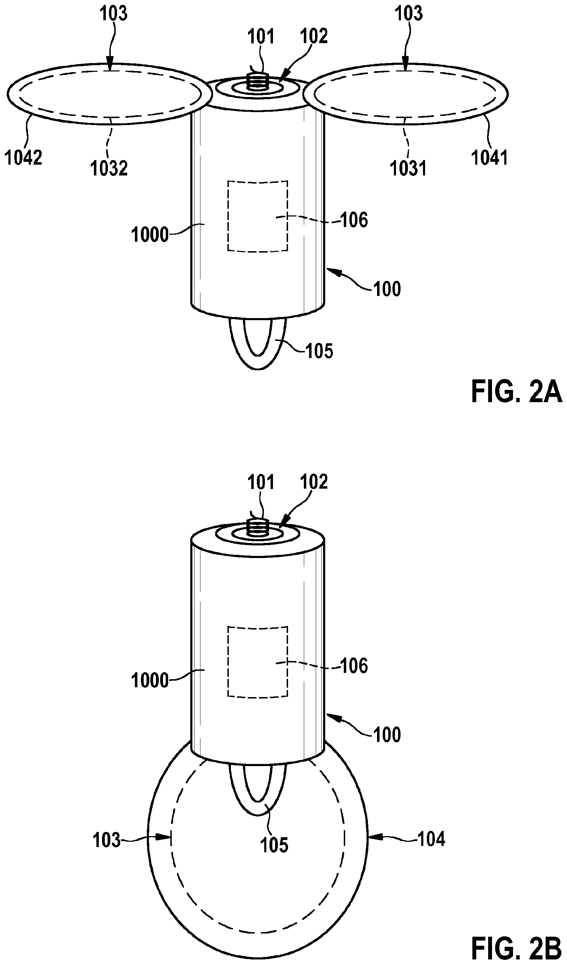

Fig. 2A schematically and exemplarily illustrates an implantable sensor device 100, such as the one of Fig. 1, in some more detail: The sensor device 100 comprises a hermetically sealed housing 1000 having a cylindrical shape. A digital processing device 106, such as a processor unit and a memory unit, is contained in the housing 1000. Further, at least a part of the communication system 103, such as transmitter and/or receiver unit for unidirectional or bidirectional communication, as well as a battery of the sensor device 100 may be included in the hermetically sealed housing 1000 (not illustrated).

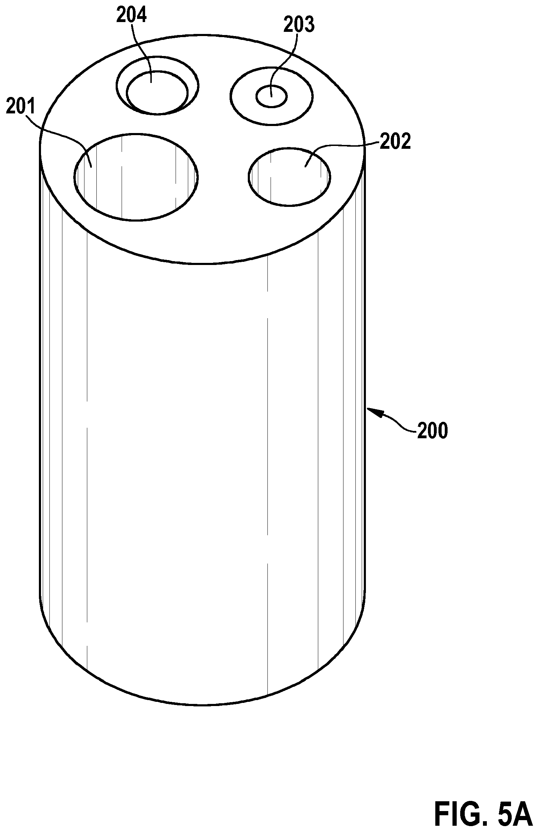

The housing 1000 may comprise or consists of a biocompatible material, such as titanium, PEEK, polyurethane, platinum, silicone, tantalum, or MP35N. A maximum outside diameter of the housing 1000 transverse to its longitudinal cylinder axis may be in the range from 3 mm to 15 mm, for example. The sensor device 1000 may thus be suitable to be guided through a working channel 201, 202 of a commercial gastroscope 200 or to be attached distally to a gastroscope 200 without increasing the outside diameter of the gastroscope arrangement. This will be further explained below with reference to Figs. 5A-D.

A contact element 102 in the form of an annular metal surface is arranged at one end of the housing 1000. The contact element 102 forms contact electrode for contacting the stomach wall 500, 502 and for receiving electrical signals from the patient's heart 400.

In the present embodiment, the fixing element 101 is helical screw which may is arranged at the same end of such a cylindrical housing 1000 as the annular contact element 102. For example, the fixing element 101 may be arranged in the center of the annular contact element 102, as illustrated. The fixing element 101 is provided for attaching the sensor device 100 to the stomach wall 500. At the same time, the fixing element 101 may serve as a second electrode pole (i. e., as a second contact element) for receiving the electrical signals from the heart 400 via the stomach wall 500. In other words, the annular contact element 102 and the fixing element 101 together may form a pair of contact electrodes 101, 102 for receiving electrical heart signals.

Further, in the exemplary embodiment of Fig. 2A, the communication system 103 comprises two loop antennas 1031, 1032 which are arranged at the end of the cylindrical housing 1000 that is in contact with the stomach wall 500, 502, i. e., at the same end of the housing 1000 as the fixing element 101 and the annular contact element 102.

Each of the loop antennas 1031, 1032 is embedded in a respective protective plastic sheath 1041, 1042. The protective plastic sheaths 1041, 1042 may be flexible. For example, the protective plastic sheaths 1041, 1042 may comprise of consist of silicone or of another flexible plastic material.

At the other end of the cylindrical housing 1000, a rigid loop 105 is attached. For example, the sensor device 100 may be rotated with the help of the rigid loop 105, e. g., for fixing the sensor device 100 and/or for facilitating a replacement of its battery.
Fig. 2B shows another exemplary embodiment of the sensor device 100. The basic structure of the sensor device 100 according to this embodiment is similar to the embodiment of Fig. 2A, the only difference being the arrangement of the communication antenna system 103: In this embodiment, a loop antenna 103 is arranged at the other end of the housing 1000, i. e., opposite to the end that is in contact with the stomach wall 500, 502.

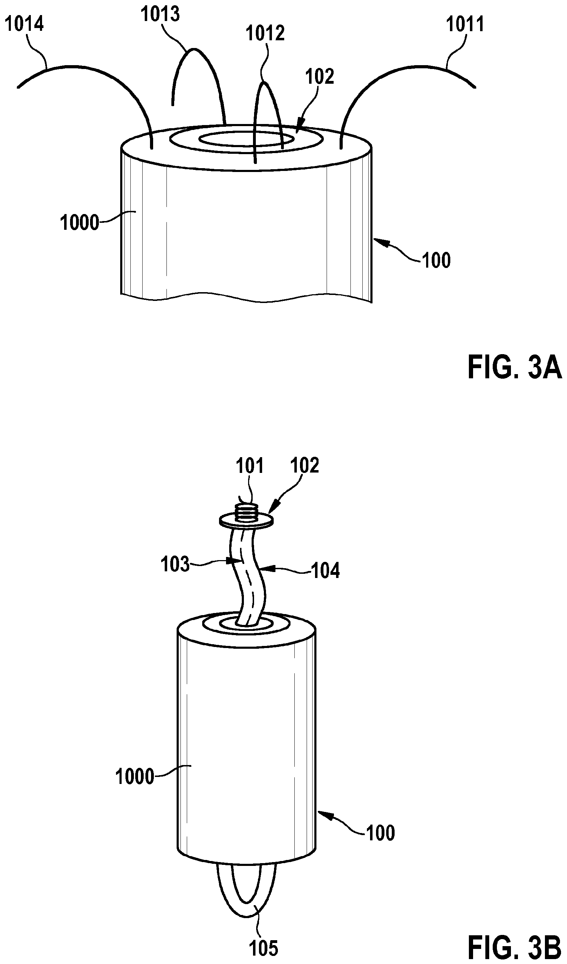

Fig. 3A schematically and exemplarily illustrated a further embodiment of the sensor device 100, wherein the fixing element 101 is provided in the form of a plurality of hooks 1011, 1012, 1013, 1014. For example, the hooks 1011, 1012, 1013, 1014 may comprise or consist of a shape memory material, such as nitinol. The fixing hooks 1011, 1012, 1013, 1014 may thus be configured for forming an arch, i. e., the hook shape illustrated in Fig. 3A, when they pushed out of a lumen. For example, the fixing hooks 1011, 1012, 1013, 1014 (e. g., together with the sensor device 100 as a whole) may be pushed out of a working channel 201, 202 of a gastroscope 200, cf. Figs. 5A-D). Conversely, the hook-shaped fixing elements 1011, 1012, 1013, 1014 may be retractable into a lumen, thereby assuming a more elongate shape.

In a further variant embodiment of the sensor device 100, which is schematically and exemplarily illustrated in Fig. 3B, the fixing element 101 is arranged at an end of an elongate electrode element, e. g. in the form a screw. For example, the elongate electrode element may form part of the communication system 103 and may be used as a monopolar antenna (or a part thereof). The antenna 103 may be embedded in a flexible plastic sheath 104. Also the contact element 102 may be arranged at the end of the elongate electrode element 103, as exemplarily illustrated in Fig. 3B.

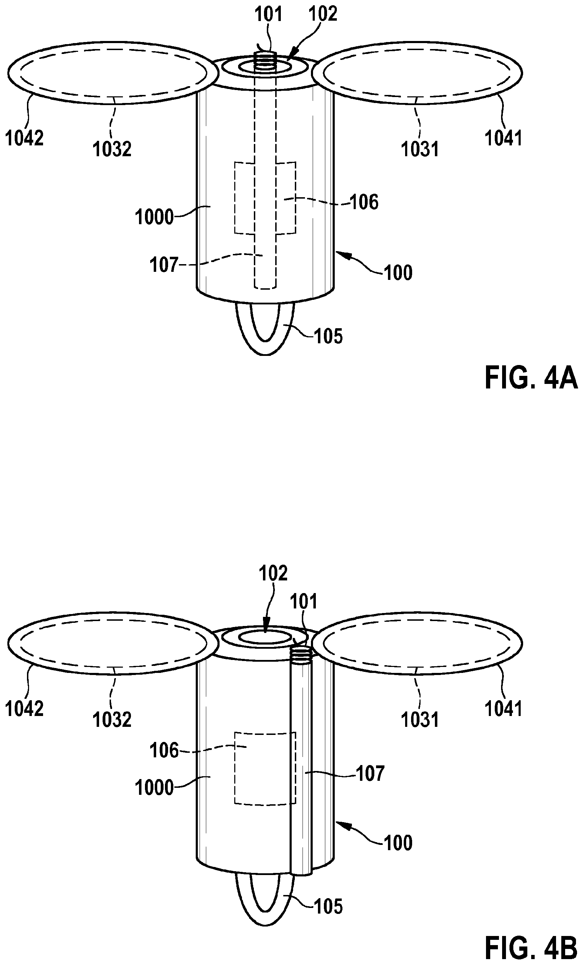

Figs. 4A-B schematically and exemplarily show further embodiments of the sensor device 100. In these embodiments, the fixing element 101 is a rotatable screw 101 extending at least partially in a lumen 107 provided in the sensor device 100. For example, the screw 101 may be rotated (screwed) by means of a screw mandrin so as to protrude from the housing 1000 and engage with the stomach wall 500, 502 when the sensor device 100 has reached a suitable position. To this end, the screw mandrin may be inserted in the lumen 107 where it may engage with the rotatable screw.

The lumen 107 and the fixing screw 101 may be guided centrally or decentrally within the housing 1000, as illustrated in Fig. 4A, or may be arranged externally on the housing 100, as illustrated in Fig. 4B. In the case of a decentralized screw fixation, for example, a contact element 102 in the form of a metal surface being arranged next to the fixing screw 101 can be used as a second electrode pole (in addition to the fixing screw 101) for the detection of the cardiac excitation signals.

Fig. 5A schematically and exemplarily illustrates a gastroscope 200 which may be used for implanting the sensor device 100 in accordance with one or more embodiments. For example, as usual for commercially available gastroscopes for diagnosis or interventions, the gastroscope may comprise a light source 204, a camera or a CCD sensor 203, and one or more working channels 201, 202. The working channels 201, 202 may have a diameter in the range from, e. g., 2 mm to 15 mm. The outside diameter of the gastroscope 200 may be in the range from 5 mm to 20 mm, for example.

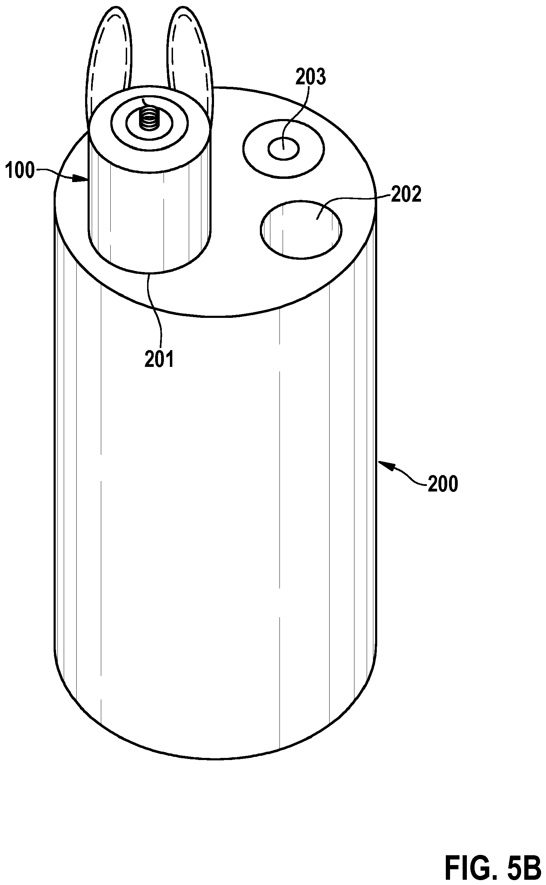

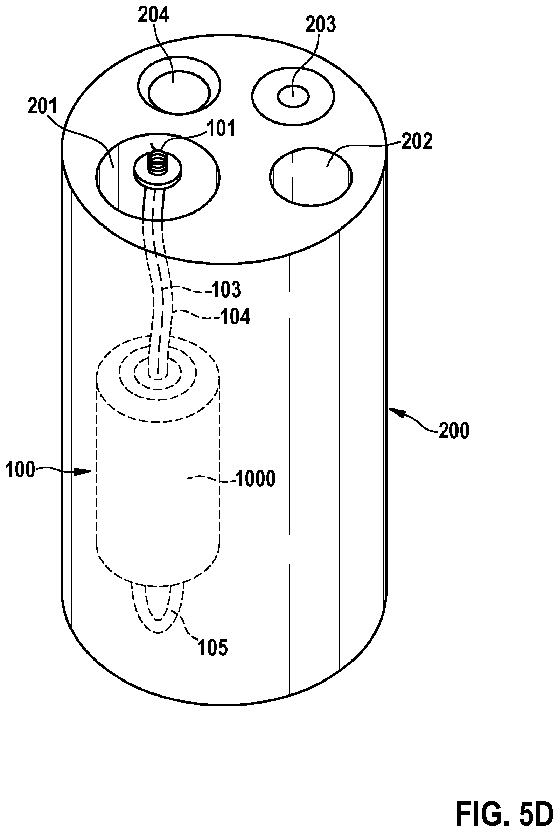

For example, the sensor device 100 may be arranged inside a working channel 201 of the gastroscope 200 when passing through the esophagus 800, as schematically illustrated in each of Figs. 5B and 5D. In particular, the sensor device 100 may be entirely contained in the working channel 201 during this phase of the implantation. This variant may be appropriate especially in case the fixing element 101 is a fixed screw protruding from the housing 1000 of the sensor device 100, because injuries of the esophagus 800 may thus be prevented.

A diameter of the sensor device 100 may be in the range from, e. g., 3 mm to 5 mm, so that the sensor device 100 can be readily pushed through the working channel 201. When the sensor device 100 has reached a suitable position in the gastric fundus 501, it may be fastened by means of the fixing element 101, e. g., by rotating the sensor device 100, thereby screwing the fixing element 101 into the stomach wall 500, 502.

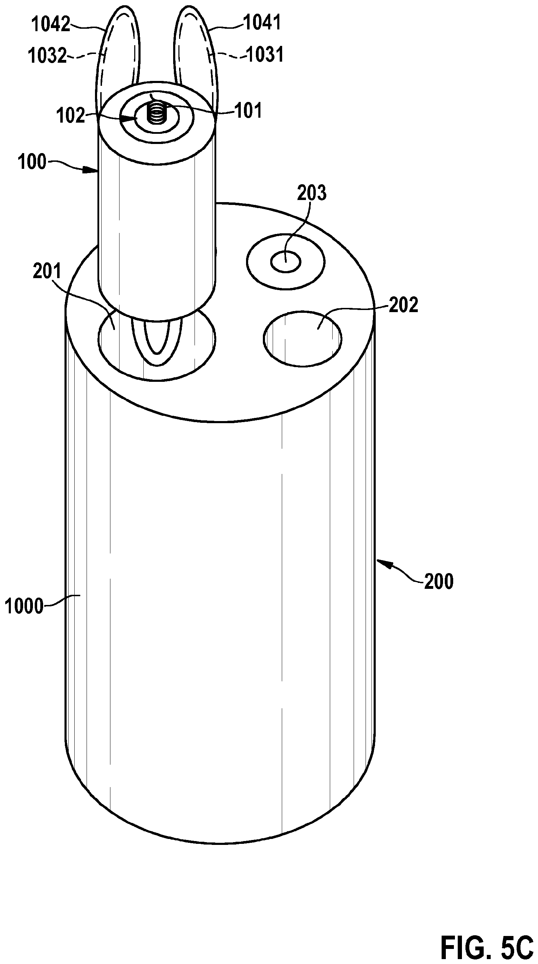

Alternatively, as schematically and exemplarily illustrated in Fig. 5C, the sensor device 100 may be attached at a distal end of the gastroscope 200 when passing through the esophagus 800. This is to say that the sensor device 100 may also be arranged external of the working channel(s) 201, 202 of the gastroscope 200 during this phase of the implantation. In this case, a larger diameter of the sensor device 100, e. g. similar to the outer diameter of the gastroscope 200, is possible. The attachment of the sensor device 100 outside the gastroscope 200 may be effected using a clamping instrument (such as a biopsy forceps) inside the working channel 201. For example, the clamping instrument may be connected to the rigid loop 105 arranged on the housing 1000 of the sensor device 100.

As exemplarily and schematically illustrated, for example, in Fig. 5C, the sensor device 100 may be configured for assuming a transport configuration, in which the antenna 103, 1031, 1032 and the protective plastic sheath 104, 1041, 1042 are folded over the fixing element 101. Thus, the risk of injury, e. g., of the esophagus 800, during implantation may be minimized. This may be particularly advantageous in embodiments wherein the fixing element is a fixed screw 101 protruding from the housing 1000.

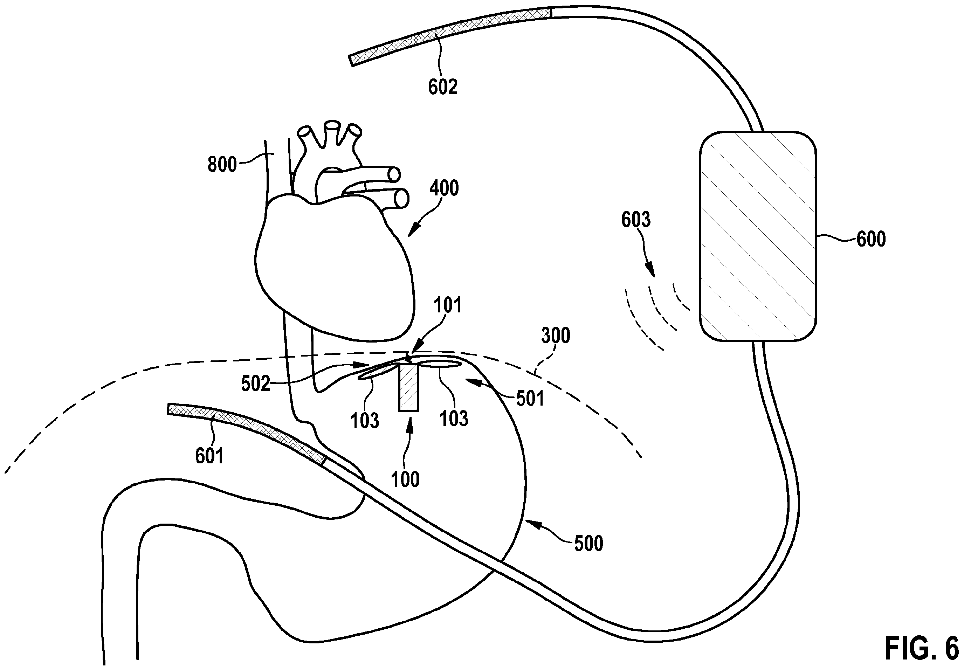

Fig. 6 schematically and exemplarily illustrated a system 700 comprising a subcutaneous implantable cardioverter-defibrillator (sICD) 600, which comprises a pulse generator and sICD electrodes 601, 602, and a sensor device 100 as described above. It should be noted that the sICD 600 including the sICD electrodes 601, 602 is depicted only schematically, i. e., Fig. 6 does not reflect a correct positioning of the sICD 600 within the patient's body.

The sensor device 100 is configured for delivering electrical pulses to the stomach wall 500, 502 in dependence on an sICD pulse delivered by the sICD 600. For example, the electrical pulses may be delivered by the sensor device via the contact element 102 and/or via a further contact element (e. g., the fixing element 101) that is adapted to be arranged in contact with the stomach wall 500, 502. For example, the fixing element 101 may at the same time act as a contact element for delivering the electrical pulses.

For example, the sensor device 100 may detect an electric field resulting from the one or more sICD pulses and deliver the one or more electrical pulses in reaction to the detecting this electric field. Additionally or alternatively, the sICD 600 may inform the sensor device 100 via a communication link 603 about the delivery of the one or more sICD pulses and the sensor device 100 may deliver the one or more electrical pulses in reaction to being informed about the delivery of the one or more sICD pulses via the communication link 603.

Thus, the sICD 600 may inform the sensor device 100 when a shock has been triggered and, as a reaction, the sensor device 100 may start a stimulation. Alternatively or additionally, the sensor device 100 may detect the strongly increased electric fields that arise during a shock and may then initiate an after-shock pacing on its own.

### List of reference numerals

- 100: Sensor device
- 1000: Housing
- 101: Fixing element
- 1011: Fixing hook
- 1012: Fixing hook
- 1013: Fixing hook
- 1014: Fixing hook
- 102: Contact element
- 103: Communication system
- 1031: Antenna loop
- 1032: Antenna loop
- 104: Plastic sheath
- 1041: Plastic sheath
- 1042: Plastic sheath
- 105: Rigid loop
- 200: Gastroscope
- 201: Working channel
- 202: Working channel
- 203: Camera / CCD sensor
- 204: Light source
- 300: Thoracic diaphragm
- 400: Heart
- 500: Stomach wall
- 501: Gastric fundus
- 502: Gastric fornix
- 600: Subcutaneous implantable cardioverter-defibrillator (sICD)
- 601: sICD electrode
- 602: sICD electrode
- 603: Communication link
- 700: System
- 800: Esophagus

## Claims

1. A sensor device (100) adapted to be implanted in the gastric fundus (501) of a patient, the sensor device (100) comprising:
- a fixing element (101) configured for fixing the sensor device (1) to the stomach wall (500, 502);
- a contact element (101, 102) adapted to be arranged in contact with the stomach wall (500, 502) and to receive electricals signals from the patient's heart (400) via the stomach wall (500, 502);
- a digital processing device (106) configured for processing the received electrical signals (400); and
- a communication system (103) configured for wirelessly transmitting signals to a receiver device in dependence on the processed electrical signal.

2. The sensor device (100) according to claim 1, wherein the fixing element (101) comprises at least one of a screw and a hook.

3. The sensor device (100) according to one of the preceding claims, wherein the fixing element (101) is arranged at an end of an elongate electrode element (102, 103, 104).

4. The sensor device (100) according to one of the preceding claims, wherein the sensor device (100) comprises a hermetically sealed housing (1000), wherein the digital processing device (106) and at least a part of the communication system (103) are arranged inside the housing (1000).

5. The sensor device (100) according to one of the preceding claims, wherein the fixing element (101) is a rotatable screw extending at least partially in a lumen (107) provided in the sensor device (100).

6. The sensor device (100) according to one of the preceding claims, wherein the communication system (103) comprises at least one of a loop antenna, a dipolar antenna, and a monopolar antenna.

7. The sensor device (100) according to claim 6, wherein the antenna (103) is embedded in a protective plastic sheath (104).

8. The sensor device (100) according to claim 7, wherein the sensor device (100) is configured for assuming a transport configuration, in which the antenna (103) and the protective plastic sheath are folded over the fixing element (101).

9. The sensor device (100) according to one of the preceding claims, wherein the sensor device (100) is configured for delivering electrical pulses to the stomach wall (500, 502) via the contact element (101, 102) and/or a further contact element that is adapted to be arranged in contact with the stomach wall (500, 502).

10. A system (700), comprising:
- a subcutaneous implantable cardioverter-defibrillator (sICD) (600); and
- a sensor device (100) according to claim 9,
wherein the sensor device (100) is configured for delivering one or more electrical pulses to the stomach wall in dependence on an sICD pulse delivered by the sICD (600).

11. The system (700) of claim 10, wherein the sensor device (100) is communicatively coupled to the sICD (600) via a communication link (603).

12. A method for monitoring a cardiac activity of a patient, comprising:
- providing a sensor device (100) according to one of claims 1 to 9;
- implanting the sensor device (100) in the gastric fundus (501) of the patient via the esophagus (800); and
- receiving, by means of a receiver device, signals transmitted by the communication system (103), the transmitted signals being indicative of the cardiac activity.

13. The method of claim 12, wherein the sensor device (100) is implanted by means of a gastroscope (200), wherein the sensor device (100) is arranged inside a working channel (201) of the gastroscope (200) when passing through the esophagus (800).

14. The method of claim 12, wherein the sensor device (100) is implanted by means of a gastroscope (200), wherein the sensor device (100) is attached at a distal end of the gastroscope (200) when passing through the esophagus (800).

15. The method of one of claims 12 to 14, wherein the method comprises:
- providing a system (700) according to one of claims 10 or 11;
- implanting each of the sensor device (100) and the sICD (600) in the patient's body, wherein the sensor device (100) is implanted in the gastric fundus (501) via the esophagus (800);
- delivering one or more sICD pulses by means of the sICD (600); and
- delivering one or more electrical pulses to the stomach wall (500, 502) by means of the sensor device (100) in dependence on the one or more sICD pulses delivered by the sICD (600).
